# EUROPÄISCHE PATENTSCHRIFT

(11) **EP 2 558 472 B1**
(45) Veröffentlichungstag und Bekanntmachung des Hinweises auf die Patenterteilung: **13.08.2014**
(21) Anmeldenummer: 11715193.6
(22) Anmeldetag: 08.04.2011
(51) Int. Cl.: C07D 495/04

(54) **VERFAHREN ZUR HERSTELLUNG VON DITHIIN-TETRACARBOXY-DIIMIDEN**
METHOD FOR PRODUCING DITHIIN TETRACARBOXY-DIIMIDES
PROCÉDÉ DE FABRICATION DE DITHIINE-TÉTRACARBOXY-DIIMIDES

(30) Priorität: 16.04.2010 US 325081 P; 14.04.2010 EP 10159900
(43) Veröffentlichungstag der Anmeldung: 20.02.2013
(73) Patentinhaber: Bayer CropScience AG, 40789 Monheim (DE)
(72) Erfinder: HIMMLER, Thomas, 51519 Odenthal (DE)
(74) Vertreter: BIP Patents
(86) Internationale Anmeldenummer: PCT/EP2011/055513
(87) Internationale Veröffentlichungsnummer: WO 2011/128264

(56) Entgegenhaltungen:
- US-A- 3 364 229

## Beschreibung

Die vorliegende Erfindung betrifft ein neues Verfahren zur Herstellung von Dithiin-tetracarboxy-diimiden.

Dithiin-tetracarboxy-diimide als solche sind bereits bekannt. Ebenso ist bekannt, dass diese Dithiin-tetracarboxy-diimide als Anthelminthika gegen innere Parasiten von Tieren, insbesondere Nematoden, verwendet werden können und insektizide Wirkung aufweisen (vgl. US 3,364,229). Außerdem ist bekannt, dass bestimmte Dithiin-tetracarboxy-diimide antibakterielle Wirkung besitzen und gegen menschliche Mykosen eine gewisse Wirkung aufweisen (vgl. Il Farmaco 2005, 60, 944-947). Weiterhin ist bekannt, dass Dithiin-tetracarboxy-diimide als Pigmente in elektrophotographischen Photorezeptoren oder als Farbstoffe in Lacken und Polymeren eingesetzt werden können (vgl. JP-A 10-251265, PL-B 143804).

### Dithiin-tetracarboximide der Formel (I)

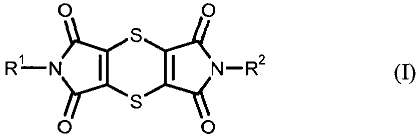

in welcher
- R¹ und R²: gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) stehen,
- R³: für Wasserstoff, C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
- R⁴: für Hydroxy, C₁-C₄-Alkyl oder C₁-C₄-Alkoxy steht,
können auf verschiedene bekannte Weisen hergestellt werden.

Beispielsweise wird in einem bekannten Verfahren (vgl. Synthetic Communications 2006, 36, 3591-3597) in einer ersten Stufe Bernsteinsäureanhydrid mit einem Amin der Formel (II) gegebenenfalls in Gegenwart eines Verdünndungsmittels umgesetzt. Anschließend werden dann die so erhaltenen Bemsteinsäuremonoamide der Formel (III) mit einem großen Überschuss an Thionylchlorid in Gegenwart von Dioxan als Verdünnungsmittel bei Raumtemperatur umgesetzt, wobei in einer Abfolge zahlreicher Reaktionsschritte schließlich die Dithiin-tetracarboxy-diimide der Formel (I) erhalten werden. Die Dithiin-tetracarboxy-diimide werden wahlweise direkt aus dem Reaktionsgemisch oder nach Versetzen mit Wasser durch Filtration isoliert. Je nach Reaktionsbedingungen (Verdünnungsmittel) und Art der Reste R können unter Umständen die Dithiin-diisoimide der Formel (IV) isoliert werden, bevor man sie in die Dithiin-tetracarboxy-diimide der Formel (I) überführt:

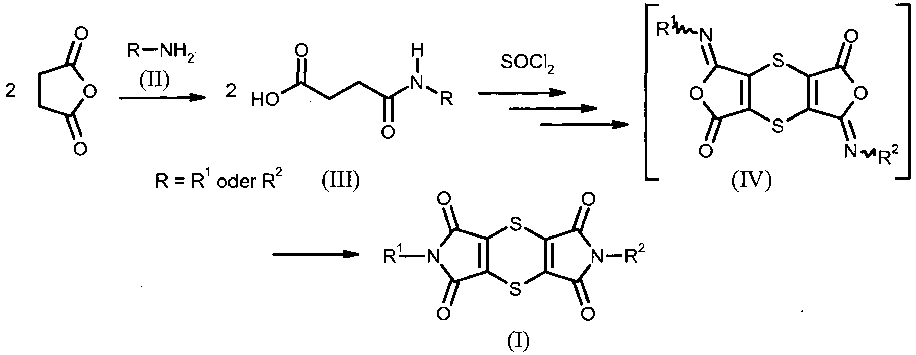

Nachteilig an diesem Verfahren sind die langen Reaktionszeit sowie das Ergebnis, dass entweder die erhaltenen Ausbeuten in der Regel etwa 30-40% der Theorie nicht überschreiten oder aber die Reinheiten der isolierten Produkte ungenügend sind. Außerdem ist bei einer wässrigen Aufarbeitung des Reaktionsgemisches nachteilig, dass dabei große Mengen Thionylchlorid vernichtet werden; die entstehenden Gase (SO₂ und HCl) müssen entsorgt werden. Ebenfalls nachteilig ist der Umstand, dass erfahrungsgemäß das Produkt nicht in einer Portion erhalten wird. Vielmehr ist es häufig so, dass nach einer ersten Produktisolierung durch Filtration aus dem Filtrat nach längerem Stehen (beispielsweise über Nacht), weiteres Produkt ausfällt, das erneut durch Filtration isoliert werden muss. Zuweilen muss dieser Vorgang nochmals durchgeführt werden. Diese Arbeitsweise ist sehr umständlich und zeitraubend.

In einem weiteren bekannten Verfahren (vgl. US 3,364,229; Chem. Ber. 1967, 100, 1559-70) wird in einer ersten Stufe Dichlormaleinsäureanhydrid der Formel (V) mit einem Amin der Formel (II) gegebenenfalls in Gegenwart eines Verdünndungsmittels umgesetzt. Anschließend werden dann die so erhaltenen Dichlor-maleinsäureimide der Formel (VI) mit einer Schwefel-freisetzenden Verbindung (beispielsweise Schwefelwasserstoff, Thioharnstoff oder Natriumthiosulfat) umgesetzt:

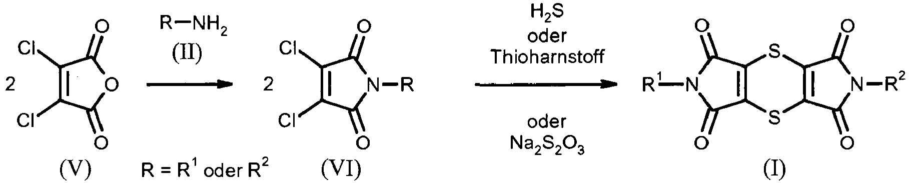

Dieses Verfahren hat den Nachteil, dass beispielsweise der Umgang mit dem hochgiftigen Schwefelwasserstoffgas technisch sehr schwierig und aufwendig ist. Bei der Verwendung von Thioharnstoff werden neben dem Zielprodukt unerwünschte Nebenprodukte erhalten, die nur sehr schwierig zu entfernen sind und die erreichbaren Ausbeuten verschlechtern. Bei der Verwendung von Natriumthiosulfat ist die beschriebene Ausbeute für einen technischen Prozess ungenügend.

Es besteht demnach weiterhin Bedarf an einem technisch einfachen und ökonomischen Herstellverfahren für Dithiin-tetracarboxy-diimide der Formel (I).

Es wurde ein neues Verfahren zum Herstellen von Dithiin-tetracarboxy-diimiden der allgemeinen Formel (I) 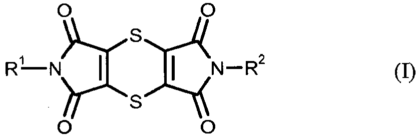 in welcher R¹ und R² die oben angegebenen Bedeutungen haben,
gefunden, dadurch gekennzeichnet, dass man

### Dichlormaleinsäureimide der Formel (VI)

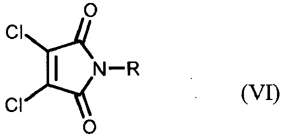

in welcher R für R¹ oder R² steht,
mit einem anorganischen Thiosulfat in einem Lösungsmittel oder Lösungsmittelgemisch in einem Molverhältnis zwischen 1,3 und 1,6 Mol Thiosulfat pro Mol Dichlormaleinsäureimid der Formel (VI) umsetzt.

Die bei der Durchführung des erfindungsgemäßen Verfahrens als Ausgangstoffe eingesetzten Dichlormaleinsäureimide sind durch die Formel (VI) allgemein definiert. R steht für die Bedeutungen von R¹ oder R².
- R¹ und R²: sind bevorzugt gleich oder verschieden und stehen bevorzugt für Wasserstoff, für gegebenenfalls einfach oder mehrfach durch Fluor, Chlor, Brom, -OR³, -COR⁴ substituiertes C₁-C₆-Alkyl, gegebenenfalls einfach oder mehrfach durch Chlor, Methyl oder Trifluormethyl substituiertes C₃-C₇-Cycloalkyl, für jeweils gegebenenfalls einfach oder mehrfach durch Fluor, Chlor, Brom, Methyl, Trifluormethyl, -COR⁴, Sulfonylamino substituiertes Phenyl oder Phenyl-(C₁-C₄-alkyl).
- R¹ und R²: sind besonders bevorzugt gleich oder verschieden und stehen besonders bevorzugt für Wasserstoff, für gegebenenfalls einfach oder mehrfach durch Fluor, Chlor, Hydroxy, Methoxy, Ethoxy, Methylcarbonyloxy, Carboxyl substituiertes C₁-C₄-Alkyl, gegebenenfalls einfach oder mehrfach durch Chlor, Methyl oder Trifluormethyl substituiertes C₃-C₇-Cycloalkyl, für jeweils gegebenenfalls einfach bis dreifach durch Fluor, Chlor, Brom, Methyl, Trifluormethyl, -COR⁴, Sulfonylamino substituiertes Phenyl, Benzyl, 1-Phenethyl, 2-Phenethyl oder 2-Methyl-2-phenethyl.
- R¹ und R²: sind ganz besonders bevorzugt gleich oder verschieden und stehen ganz besonders bevorzugt für Wasserstoff, Methyl, Ethyl, n-Propyl, Isopropyl, 2,2-Difluorethyl, 2,2,2-Trifluorethyl, jeweils gegebenenfalls durch Chlor, Methyl oder Trifluormethyl substituiertes Cyclopropyl oder Cyclohexyl.
- R¹ und R²: stehen insbesondere bevorzugt gleichzeitig für Methyl.
- R³: steht bevorzugt für Wasserstoff, Methyl, Ethyl, Methylcarbonyl, Ethylcarbonyl oder für gegebenenfalls einfach oder mehrfach durch Fluor, Chlor, Methyl, Ethyl, n-Propyl, Isopropyl oder Trifluormethyl substituiertes Phenyl.
- R³: steht besonders bevorzugt für Wasserstoff, Methyl, Methylcarbonyl oder für Phenyl.
- R⁴: steht bevorzugt für Hydroxy, Methyl, Ethyl, Methoxy oder Ethoxy.
- R⁴: steht besonders bevorzugt für Hydroxy oder Methoxy.
Besonders bevorzugt wird N-Methyl-dichlormaleinsäureimid (VI-1), R = Me, als Ausgangsstoff eingesetzt, wodurch man als Endprodukt die Verbindung (I-1) 2,6-Dimethyl-1H,5H-[1,4]dithiino[2,3-c:5,6-c']dipyrrol-1,3,5,7(2H,6H)-tetron erhält.

Setzt man Dichlormaleinsäureimid (VI-2), R = H als Ausgangsstoff ein, erhält man die Verbindung (I-2) 1H,5H-[1,4]Dithiino[2,3-c:5,6-c']dipyrrol-1,3,5,7(2H,6H)-tetron als Endprodukt.

Als Thiosulfat können im Prinzip alle löslichen anorganischen Thiosulfatsalze eingesetzt werden, wie beispielsweise Lithiumthiosulfat, Natriumthiosulfat, Kaliumthiosulfat, Caesiumthiosulfat, Magnesiumthiosulfat oder Ammoniumthiosulfat. Bevorzugt verwendet man Natriumthiosulfat, Kaliumthiosulfat oder Ammoniumthiosulfat, besonders bevorzugt Natriumthiosulfat oder Ammoniumthiosulfat. Selbstverständlich können auch Gemische dieser Salze verwendet werden.

Mit den Begriffen "Thiosulfat" und "Thiosulfatsalz" sollen, sofern existent, auch Hydrate dieser Salze umfasst sein.

Das Thiosulfat wird in Mengen zwischen 1,3 und 1, 6 Mol pro Mol Dichlormaleinsäureimid der Formel (VI) eingesetzt.

Das Thiosulfat kann dem Reaktionsgemisch in fester Form zugesetzt werden oder als Lösung, beispielsweise in Wasser. Gegebenenfalls kann das Thiosulfat auch in flüssiger Form als Schmelze zugegeben werden. So schmilzt beispielsweise das Natriumthisosulfat-Pentahydrat zwischen 45°C und 50°C. Bevorzugt setzt man das Thiosulfat als Lösung in Wasser zu.

Die Reaktionstemperatur des erfindungsgemäßen Verfahrens kann in weiten Grenzen variiert werden und liegt zwischen 0°C und 200°C. Zur Erzielung befriedigender Raum-Zeit-Ausbeuten wird man bevorzugt bei Temperaturen zwischen 20°C und 180°C arbeiten; besonders bevorzugt zwischen 30°C und 150°C.

Die Reaktionszeit des erfindungsgemäßen Verfahrens liegt zwischen 10 Minuten und 24 Stunden. Bevorzugt arbeitet man zwischen 30 Minuten und 12 Stunden, besonders bevorzugt zwischen 1 und 6 Stunden.

Als Lösungsmittel für das erfindungsgemäßen Verfahrens eignen sich Wasser, Dimethylsulfoxid, Sulfolan, Alkohole wie Methanol, Ethanol, Propanol, Isopropanol, Butanol, Tertiärbutanol, Cyclopentanol, Cyclohexanol, Ethylenglykol, Ethylenglykol-monomethylether, Ester wie Essigsäuremethylester, Essigsäureethylester, Amide wie Formamid, N,N-Dimethylformamid; N,N-Dimethylacetamid, N-Methylpyrrolidon, Ether wie Tetrahydrofuran, 1,4-Dioxan, Nitrile wie Acetonitril, Propionitril, Butyronitril, Benzonitril, Ketone wie Aceton, Methyl-ethylketon, Methyl-isobutylketon, Pinakolon, oder Gemische dieser Verdünnungsmittel. Bevorzugt verwendet man Wasser, Dimethylsulfoxid, Methanol, Ethanol, Propanol, Isopropanol, Butanol, Tertiärbutanol, Cyclohexanol, Ethylenglykol, Essigsäure-methylester, N,N-Dimethylformamid; N,N-Dimethylacetamid, Tetrahydrofuran, 1,4-Dioxan, Acetonitril, Aceton, Methyl-ethylketon, Methyl-isobutyl-keton, oder Gemische dieser Verdünnungsmittel.

Ganz besonders bevorzugt verwendet man Gemische aus Wasser und Methanol, Ethanol, Propanol, Isopropanol, Essigsäure-methylester, Tetrahydrofuran, 1,4-Dioxan, Acetonitril oder Aceton.

Das erfindungsgemäße Verfahren wird durch folgende Beispiele veranschaulicht, ohne auf sie eingeschränkt zu sein.

### Beispiel 1

Man legt eine Lösung von 90 g [0,5 Mol] N-Methyl-dichlormaleinsäureimid (VI-1) in 875 ml Methanol bei Raumtemperatur vor und tropft innerhalb von etwa 10 Minuten eine Lösung von 110,7 g [0,7 Mol] Natriumthiosulfat in 188 ml Wasser zu, wobei die Innentemperatur bis 42°C steigt. Nach Beendigung der Zugabe erhöht man die Temperatur auf 60°C und rührt 4 Stunden bei dieser Temperatur. Danach kühlt man das Reaktionsgemisch auf 10°C ab, saugt den Feststoff ab, wäscht ihn dreimal mit je 150 ml Wasser und dann 100 ml MeOH und trocknet. Man erhält so 53,7 g grünen Feststoff, der laut HPLC-Analyse zu 98,4 Flächen-% aus der Verbindung (I-1) besteht, was einer Ausbeute von 74,9% der Theorie entspricht.

### Beispiel 2 (Vergleichsbeispiel)

Man legt eine Lösung von 18 g [0,1 Mol] N-Methyl-dichlormaleinsäureimid (VI-1) in 100 ml Methanol bei 65°C vor und tropft innerhalb von etwa 10 Minuten eine ebenfalls auf 65°C erwärmte Lösung von 29,77 g [0,12 Mol] Natriumthiosulfat-Pentahydrat in 100 ml Wasser zu. Nach Beendigung der Zugabe rührt man noch 1 Stunde bei 65°C. Danach kühlt man das Reaktionsgemisch auf 15°C ab, gibt 17 ml Wasser hinzu und rührt 15 Minuten. Danach saugt man den Feststoff ab, wäscht ihn mit 70 ml Wasser und dann 30 ml MeOH, und trocknet. Man erhält auf diese Weise 10,1 g dunkelgrünen Feststoff, der laut HPLC-Analyse gegen Referenzsubstanz zu 97,76 Gewichtsprozent aus der Verbindung (I-1) besteht, was einer Ausbeute von 70% der Theorie entspricht.

### Beispiel 3

Man legt eine Lösung von 18 g [0,1 Mol] N-Methyl-dichlormaleinsäureimid (VI-1) in 100 ml Methanol bei 65°C vor und tropft innerhalb von etwa 10 Minuten eine ebenfalls auf 65°C erwärmte Lösung von 34,73 g [0,14 Mol] Natriumthiosulfat-Pentahydrat in 100 ml Wasser zu. Nach Beendigung der Zugabe rührt man noch 1 Stunde bei 65°C. Danach kühlt man das Reaktionsgemisch auf 15°C ab, gibt 17 ml Wasser hinzu und rührt 15 Minuten. Danach saugt man den Feststoff ab, wäscht ihn mit 70 ml Wasser und dann 30 ml MeOH, und trocknet. Man erhält auf diese Weise 11,3 g dunkelgrünen Feststoff, der laut HPLC-Analyse gegen Referenzsubstanz zu 95,9 Gewichtsprozent aus der Verbindung (I-1) besteht, was einer Ausbeute von 76,8% der Theorie entspricht.

### Beispiel 4

Man legt eine Lösung von 18 g [0,1 Mol] N-Methyl-dichlormaleinsäureimid (VI-1) in 100 ml Methanol bei 65°C vor und tropft innerhalb von etwa 10 Minuten eine ebenfalls auf 65°C erwärmte Lösung von 37,22 g [0,15 Mol] Natriumthiosulfat-Pentahydrat in 100 ml Wasser zu. Nach Beendigung der Zugabe rührt man noch 1 Stunde bei 65°C. Danach kühlt man das Reaktionsgemisch auf 15°C ab, gibt 17 ml Wasser hinzu und rührt 15 Minuten. Danach saugt man den Feststoff ab, wäscht ihn mit 70 ml Wasser und dann 30 ml MeOH, und trocknet. Man erhält auf diese Weise 10,65 g dunkelgrünen Feststoff, der laut HPLC-Analyse gegen Referenzsubstanz zu 98 Gewichtsprozent aus der Verbindung (I-1) besteht, was einer Ausbeute von 73,9% der Theorie entspricht.

### Beispiel 5

Man legt eine Lösung von 18 g [0,1 Mol] N-Methyl-dichlormaleinsäureimid (VI-1) in 100 ml Methanol bei 65°C vor und tropft innerhalb von etwa 10 Minuten eine ebenfalls auf 65°C erwärmte Lösung von 39,7 g [0,16 Mol] Natriumthiosulfat-Pentahydrat in 100 ml Wasser zu. Nach Beendigung der Zugabe rührt man noch 1 Stunde bei 65°C. Danach kühlt man das Reaktionsgemisch auf 15°C ab, gibt 17 ml Wasser hinzu und rührt 15 Minuten. Danach saugt man den Feststoff ab, wäscht ihn mit 70 ml Wasser und dann 30 ml MeOH, und trocknet. Man erhält auf diese Weise 10,4 g dunkelgrünen Feststoff, der laut HPLC-Analyse gegen Referenzsubstanz zu 97,3 Gewichtsprozent aus der Verbindung (I-1) besteht, was einer Ausbeute von 71,7% der Theorie entspricht.

### Beispiel 6

Man legt eine Lösung von 360 g [2 Mol] N-Methyl-dichlormaleinsäureimid (VI-1) in 3500 ml Methanol bei Raumtemperatur vor und tropft innerhalb von etwa 20 Minuten eine Lösung von 442,8 g [2,8 Mol] Natriumthiosulfat in 750 ml Wasser zu. Nach Beendigung der Zugabe erwärmt man auf 60°C und rührt dann noch für 4 Stunde bei dieser Temperatur. Danach kühlt man das Reaktionsgemisch auf 15°C ab, gibt 250 ml Wasser hinzu und rührt 10 Minuten. Danach saugt man den Feststoff ab, wäscht ihn mit 1200 ml Wasser und dann 600 ml MeOH, und trocknet. Man erhält auf diese Weise 210,2 g dunkelgrünen Feststoff, der laut HPLC-Analyse gegen Referenzsubstanz zu 97,9 Gewichtsprozent aus der Verbindung (I-1) besteht, was einer Ausbeute von 72,9% der Theorie entspricht.

### Beispiel 7 (Vergleichsbeispiel)

Man legt eine Lösung von 7,2 g [0,04 Mol] N-Methyl-dichlormaleinsäureimid (VI-1) in 70 ml Methanol bei Raumtemperatur vor und tropft innerhalb von etwa 10 Minuten eine Lösung von 10,74 g [0,068 Mol] Natriumthiosulfat in 23 ml Wasser zu. Nach Beendigung der Zugabe erwärmt man auf 60°C und rührt dann noch für 4 Stunde bei dieser Temperatur. Danach kühlt man das Reaktionsgemisch auf 15°C ab, gibt 10 ml Wasser hinzu und rührt 10 Minuten. Danach saugt man den Feststoff ab, wäscht ihn mit 35 ml Wasser und dann 10 ml MeOH, und trocknet. Man erhält auf diese Weise 3,83 g grünen Feststoff, der laut HPLC-Analyse gegen Referenzsubstanz zu 94,7 Gewichtsprozent aus der Verbindung (I-1) besteht, was einer Ausbeute von 64,2% der Theorie entspricht.

### Beispiel 8 (Vergleichsbeispiel)

Man legt eine Lösung von 7,2 g [0,04 Mol] N-Methyl-dichlormaleinsäureimid (VI-1) in 70 ml Methanol bei Raumtemperatur vor und tropft innerhalb von etwa 10 Minuten eine Lösung von 12,64 g [0,08 Mol] Natriumthiosulfat in 27 ml Wasser zu. Nach Beendigung der Zugabe erwärmt man auf 60°C und rührt dann noch für 4 Stunde bei dieser Temperatur. Danach kühlt man das Reaktionsgemisch auf 15°C ab, gibt 10 ml Wasser hinzu und rührt 10 Minuten. Danach saugt man den Feststoff ab, wäscht ihn mit 35 ml Wasser und dann 10 ml MeOH, und trocknet. Man erhält auf diese Weise 2,5 g grünen Feststoff, der laut HPLC-Analyse zu 83,7 Flächen-% aus der Verbindung (I-1) besteht, was einer Ausbeute von 37% der Theorie entspricht.

### Beispiel 9

Man legt eine Lösung von 18 g [0,1 Mol] N-Methyl-dichlormaleinsäureimid (VI-1) in 100 ml Ethanol bei 75°C vor und tropft innerhalb von etwa 10 Minuten eine ebenfalls auf 75°C erwärmte Lösung von 34,73 g [0,14 Mol] Natriumthiosulfat-Pentahydrat in 100 ml Wasser zu. Nach Beendigung der Zugabe rührt man noch 1 Stunde bei 75°C. Danach kühlt man das Reaktionsgemisch auf 15°C ab, gibt 17 ml Wasser hinzu und rührt 15 Minuten. Danach saugt man den Feststoff ab, wäscht ihn mit 70 ml Wasser und dann 30 ml EtOH, und trocknet. Man erhält auf diese Weise 11,0 g dunkelgrünen Feststoff, der laut HPLC-Analyse gegen Referenzsubstanz zu 97 Gewichtsprozent aus der Verbindung (I-1) besteht, was einer Ausbeute von 75,5% der Theorie entspricht.

### Beispiel 10 (Vergleichsbeispiel)

Man legt eine Lösung von 7,2 g [0,04 Mol] N-Methyl-dichlormaleinsäureimid (VI-1) in 70 ml Methanol bei Raumtemperatur vor und tropft innerhalb von etwa 10 Minuten eine Lösung von 6,52 g [0,044 Mol] Ammoniumthiosulfat in 15 ml Wasser zu. Nach Beendigung der Zugabe erwärmt man auf 45°C und rührt noch 2 Stunden bei 45°C. Danach kühlt man das Reaktionsgemisch auf 10°C ab, rührt noch 10 Minuten und saugt den Feststoff dann ab, wäscht ihn mit 35 ml Wasser und dann 10 ml MeOH, und trocknet. Man erhält auf diese Weise 3,50 g dunkelgrünen Feststoff, der laut HPLC-Analyse gegen Referenzsubstanz zu 97,1 Gewichtsprozent aus der Verbindung (I-1) besteht, was einer Ausbeute von 60,2% der Theorie entspricht.

### Beispiel 11

Man legt eine Lösung von 7,2 g [0,04 Mol] N-Methyl-dichlormaleinsäureimid (VI-1) in 70 ml Methanol bei Raumtemperatur vor und tropft innerhalb von etwa 10 Minuten eine Lösung von 8,3 g [0,056 Mol] Ammoniumthiosulfat in 15 ml Wasser zu. Nach Beendigung der Zugabe erwärmt man auf 45°C und rührt noch 4 Stunden bei 45°C. Danach kühlt man das Reaktionsgemisch auf 10°C ab, rührt noch 10 Minuten und saugt den Feststoff dann ab, wäscht ihn mit 35 ml Wasser und dann 10 ml MeOH, und trocknet. Man erhält auf diese Weise 3,85 g dunkelgrünen Feststoff, der laut HPLC-Analyse gegen Referenzsubstanz zu 98,3 Gewichtsprozent aus der Verbindung (I-1) besteht, was einer Ausbeute von 67% der Theorie entspricht.

### Beispiel 12

Man legt eine Lösung von 6,64 g [0,04 Mol] Dichlormaleinsäureimid (VI-2) in 40 ml Methanol bei 65°C vor und tropft innerhalb von etwa 10 Minuten eine ebenfalls auf 65°C erwärmte Lösung von 13,89 g [0,056 Mol] Natriumthiosulfat-Pentahydrat in 40 ml Wasser zu. Nach Beendigung der Zugabe rührt man noch 1 Stunde bei 65°C. Danach kühlt man das Reaktionsgemisch auf 15°C ab, gibt 10 ml Wasser hinzu und rührt 15 Minuten. Danach saugt man den Feststoff ab, wäscht ihn mit 30 ml Wasser und dann 15 ml MeOH, und trocknet. Man erhält auf diese Weise 3,6 g dunkelgrünen Feststoff, der laut HPLC-Analyse zu 99,6 Flächen-% aus der Verbindung (I-2) besteht, was einer Ausbeute von 71 % der Theorie entspricht.

### Vergleichsbeispiel 1 (entsprechend US 3,364,229, Bsp. IX ; R = H)

Man legt eine Lösung von 18 g [0,1 Mol] N-Methyl-dichlormaleinsäureimid (VI-1) in 100 ml Ethanol bei 75°C vor und tropft innerhalb von etwa 10 Minuten eine ebenfalls auf 75°C erwärmte Lösung von 24,8 g [0,10 Mol] Natriumthiosulfat-Pentahydrat in 100 ml Wasser zu. Nach Beendigung der Zugabe rührt man noch 1 Stunde bei 75°C. Danach kühlt man das Reaktionsgemisch auf 15°C ab, gibt 17 ml Wasser hinzu und rührt 15 Minuten. Danach saugt man den Feststoff ab, wäscht ihn mit 70 ml Wasser und dann 30 ml EtOH, und trocknet. Man erhält auf diese Weise 9,1 g dunkelgrünen Feststoff, der laut HPLC-Analyse zu 94,3 Flächen-% aus der Verbindung (I-1) besteht, was einer Ausbeute von 60,8% der Theorie entspricht.

### Vergleichsbeispiel 2

Man legt eine Lösung von 18 g [0,1 Mol] N-Methyl-dichlormaleinsäureimid (VI-1) in 100 ml Methanol bei 65°C vor und tropft innerhalb von etwa 10 Minuten eine ebenfalls auf 65°C erwärmte Lösung von 24,8 g [0,10 Mol] Natriumthiosulfat-Pentahydrat in 100 ml Wasser zu. Nach Beendigung der Zugabe rührt man noch 1 Stunde bei 65°C. Danach kühlt man das Reaktionsgemisch auf 15°C ab, gibt 17 ml Wasser hinzu und rührt 15 Minuten. Danach saugt man den Feststoff ab, wäscht ihn mit 70 ml Wasser und dann 30 ml MeOH, und trocknet. Man erhält auf diese Weise 10,4 g dunkelgrünen Feststoff, der laut HPLC-Analyse gegen Referenzsubstanz zu 89,15 Gewichtsprozent aus der Verbindung (I-1) besteht, was einer Ausbeute von 65,7% der Theorie entspricht.

### Allgemeine Angaben:

HPLC-Bedingungen: Zorbax Eclipse Plus C18 4.6*50 mm 1.8µm, Eluent A: 0.1% H₃PO₄, Eluent B: Acetonitril, Gradient: 90/10, 20%/min, 5/95 (1.75), Fluss: 2 ml/min, 55°C.

## Patentansprüche

1. Verfahren zum Herstellen von Dithiin-tetracarboxy-diimiden der allgemeinen Formel (I) 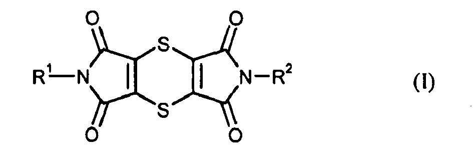 in welcher
R¹ und R² gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) stehen,
R³ für Wasserstoff, C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
R⁴ für Hydroxy, C₁-C₄-Alkyl oder C₁-C₄-Alkoxy steht,
**dadurch gekennzeichnet, dass** man
Dichlormaleinsäureimide der Formel (VI) 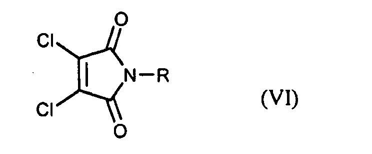 in welcher R für R¹ oder R² steht,
mit einem anorganischen Thiosulfat in einem Lösungsmittel oder Lösungsmittelgemisch in einem Molverhältnis zwischen 1,3 und 1,6 Mol Thiosulfat pro Mol Dichlormaleinsäureimid der Formel (VI) umsetzt.

2. Verfahren gemäß Anspruch 1, **dadurch gekennzeichnet, dass** man als Thiosulfat lösliche anorganische Thiosulfatsalze einsetzt, ausgewählt aus Lithiumthiosulfat, Natriumthiosulfat, Kaliumthiosulfat, Caesiumthiosulfat, Magnesiumthiosulfat oder Ammoniumthiosulfat oder Gemischen hiervon.

## Claims

1. Process for preparing dithiine-tetracarboxy-diimides of the general formula (I) 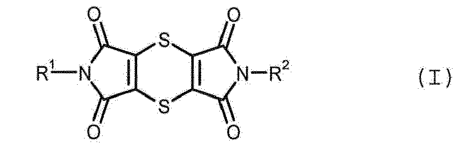 in which
R¹ and R² are identical or different and are hydrogen, or are C₁-C₈-alkyl which is optionally substituted one or more times by halogen, -OR³, and/or -COR⁴, are C₃-C₇-cycloalkyl which is optionally substituted one or more times by halogen, C₁-C₄-alkyl or C₁-C₄-haloalkyl, or are aryl or aryl-(C₁-C₄-alkyl) each of which is optionally substituted one or more times by halogen, C₁-C₄-alkyl, C₁-C₄-haloalkyl, -COR⁴ or sulphonylamino,
R³ is hydrogen, C₁-C₄-alkyl or C₁-C₄-alkylcarbonyl or is aryl which is optionally substituted one or more times by halogen, C₁-C₄-alkyl or C₁-C₄-haloalkyl,
R⁴ is hydroxyl, C₁-C₄-alkyl or C₁-C₄-alkoxy,
**characterized in that**
dichloromaleimides of the formula (VI) 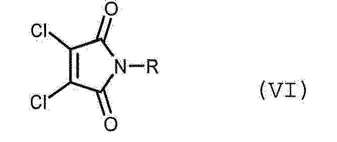 in which R is R¹ or R² are reacted with an inorganic thiosulphate in a solvent or solvent mixture in a molar ratio between 1.3 and 1.6 mol of thiosulphate per mole of dichloromaleimide of the formula (VI).

2. Process according to Claim 1, **characterized in that** the thiosulphate used is soluble inorganic thiosulphate salts selected from lithium thiosulphate, sodium thiosulphate, potassium thiosulphate, caesium thiosulphate, magnesium thiosulphate or ammonium thiosulphate or mixtures hereof.

## Revendications

1. Procédé de fabrication de dithiine-tétracarboxy-diimides de formule générale (I) 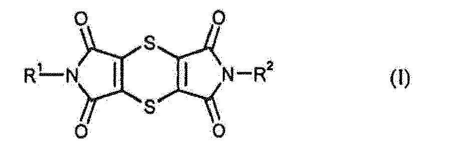 dans laquelle
R¹ et R² sont identiques ou différents et représentent hydrogène ; alkyle en C₁-C₈ éventuellement substitué une ou plusieurs fois par halogène, -OR³, -COR⁴; cycloalkyle en C₃-C₇ éventuellement substitué une ou plusieurs fois par halogène, alkyle en C₁-C₄ ou halogénoalkyle en C₁-C₄ ; aryle ou aryl-(alkyle en C₁-C₄) chacun éventuellement substitué une ou plusieurs fois par halogène, alkyle en C₁-C₄, halogénoalkyle en C₁-C₄, - COR⁴ ou sulfonylamino,
R³ représente hydrogène, alkyle en C₁-C₄, alkylcarbonyle en C₁-C₄ ; ou aryle éventuellement substitué une ou plusieurs fois par halogène, alkyle en C₁-C₄ ou halogénoalkyle en C₁-C₄,
R⁴ représente hydroxy, alkyle en C₁-C₄ ou alcoxy en C₁-C₄,
**caractérisé en ce que**
des imides de l'acide dichloromaléique de formule (VI) 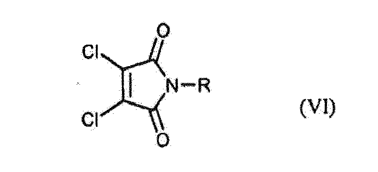 dans lesquels R représente R¹ ou R²,
sont mis en réaction avec un thiosulfate inorganique dans un solvant ou un mélange de solvants en un rapport molaire compris entre 1,3 et 1,6 mole de thiosulfate par mole d'imide de l'acide dichloromaléique de formule (VI).

2. Procédé selon la revendication 1, **caractérisé en ce que** des sels de thiosulfate inorganiques solubles sont utilisés en tant que thiosulfate, choisis parmi le thiosulfate de lithium, le thiosulfate de sodium, le thiosulfate de potassium, le thiosulfate de césium, le thiosulfate de magnésium ou le thiosulfate d'ammonium ou leurs mélanges.
